# EUROPEAN PATENT APPLICATION

(11) **EP 4 570 199 A1**
(43) Date of publication of application: **18.06.2025**
(21) Application number: 24219443.9
(22) Date of filing: 12.12.2024
(51) Int. Cl.: A61B 17/70, A61B 17/80, A61B 17/72

(54) **EXPANDABLE BONE IMPLANT SYSTEM**

(30) Priority: 14.12.2023 US 202363609932 P
(71) Applicant: Stryker European Operations Limited, Carrigtwohill T45H X08 (IE)
(72) Inventor: HEEDE, Andreas, 24539 Neumünster (DE)
(74) Representative: Maiwald GmbH

(57) **Abstract**

An expandable bone implant system comprises a first body defining an internal cavity, a perforated cap configured to attach to a first side of the first body to enclose the internal cavity, a semi-permeable member disposed at least partially between the cap and the internal cavity, a second body having a sealing end positioned within the internal cavity of the first body, a free end extending from a second side of the first body, the second body being moveable relative to the first body, and a restrictor controlling fluid flow through the perforated cap. The first body includes an osmotic chamber defined within the internal cavity which is fluidly connected to the semi-permeable member such that fluid flow through the cap from the semi-permeable member to the osmotic chamber in a first direction facilitates movement of the second body in the first direction relative to the first body.

## Description

### BACKGROUND OF THE INVENTION

This application claims the benefit of the filing date of United States Provisional Application No. 63/609,932 filed December 14, 2023.

### BACKGROUND OF THE INVENTION

The present disclosure relates to an expandable bone implant system that is capable of expansion for use in treating a variety of bone deformities.

There are many instances in treating orthopedic conditions where having a device that can alter or vary its size could be beneficial. For instance, in pediatric patients, devices are often implanted to correct the orthopedic condition and remain implanted for a period of time to maintain the corrected position. However, as the pediatric patient grows, it is desirable for the implanted device to grow with the patient to allow proper development of the bones.

Existing expandable bone implant systems typically rely upon intricate actuation means, such as the use of magnets and motors to cause distraction of separate plate or rod portions to lengthen the overall implant. Alternatively, expandable bone implant systems may be mechanically distractible plates or rods that employ manually movable plates or rods with the use of a distraction instrument. However, these expandable bone implant systems require numerous subsequent invasive surgical interventions to lengthen the plates or rods as the patient grows.

Thus, improvements for expandable devices that can be used for predictable distraction over time are needed.

### BRIEF SUMMARY OF THE INVENTION

A first aspect of the present disclosure includes an expandable bone implant system comprising a first body, a perforated cap, a semi-permeable member, a second body, and a restrictor. The first body defines an internal cavity. The perforated cap has perforations and is configured to attach to a first side of the first body. The semi-permeable member is disposed adjacent to the perforations of the perforated cap. The second body has a sealing end positioned within the internal cavity of the first body and a free end extending from a second side of the first body, the second body being moveable relative to the first body. The restrictor is configured to control fluid flow through the perforations of the perforated cap. The first body includes an osmotic chamber defined within the internal cavity, and the osmotic chamber is fluidly connected to the semi-permeable member such that fluid flow through the cap from the semi-permeable member to the osmotic chamber in a first direction facilitates movement of the second body in the first direction relative to the first body.

In another embodiment of the first aspect, the restrictor is positioned on the first side of the first body over the perforated cap. The restrictor is rotatable about an axis parallel to the first direction. The restrictor includes perforations configured to align with perforations of the perforated cap. The restrictor is configured to be aligned in a first orientation with respect to the perforated cap in which substantially all of a total cross-sectional area of the perforations of the perforated cap is exposed to fluid flow, in a second orientation with respect to the perforated cap in which the restrictor at least partially blocks the perforations of the perforated cap such that less than the total cross-sectional area of the perforations of the perforated cap is exposed to fluid flow, and in a third orientation with respect to the perforated cap in which the restrictor substantially blocks the perforations of the perforated cap such that the total cross-sectional area of the perforations of the perforated cap is substantially blocked to fluid flow.

In yet another embodiment of the first aspect, the restrictor defines an indent in a peripheral edge configured to provide access for a tool to rotate the restrictor with respect to the perforated cap. In yet another embodiment of the first aspect, the restrictor includes a projection disposed within a recess of the perforated cap, the projection having a width smaller than a width of the recess such that the projection can be moved between ends of the recess to limit rotation of the restrictor with respect to the perforated cap.

In yet another embodiment of the first aspect, the restrictor is moveable in the first direction and in a second direction opposite the first direction. The restrictor includes a plurality of plugs respectively aligned with perforations of the perforated cap. The restrictor is configured to be translated from a first orientation with respect to the perforated cap in which the perforations of the perforated cap are exposed to fluid flow to a second orientation with respect to the perforated cap in which the plurality of plugs are at least partially inserted into the perforations, respectively, to substantially block the perforations of the perforated cap from fluid flow.

In yet another embodiment of the first aspect, the first body further includes a valve configured to release pressure from the osmotic chamber. In yet another embodiment of the first aspect, the first body includes a first plate portion and the second body includes a second plate portion, the first and second plate portions separated by a gap located adjacent the second side of the first body. The first and second plate portions each include apertures through which fasteners can be inserted to secure the first and second plate portions to respective bone portions.

A second aspect of the present disclosure includes a method for expanding a bone comprising anchoring a first body of an expandable bone implant system on the bone, anchoring a second body on the same bone, and cutting the bone. The first body defining an internal cavity and including an osmotic chamber defined within the internal cavity. The system further including a perforated cap configured to attach to a first side of the first body to enclose the internal cavity and a semi-permeable member disposed between the cap and the internal cavity. The second body having a sealing end positioned within the internal cavity of the first body and a free end extending from a second side of the first body, the second body being moveable relative to the first body. After the anchoring steps, cutting the bone at the second side of the first body such that a first portion of the bone is connected to the first body and a separated second portion of the bone is connected to the second body, wherein the osmotic chamber is fluidly connected to the semi-permeable member such that fluid flow through the cap from the semi-permeable member to the osmotic chamber in a first direction facilitates movement of the second body in the first direction relative to the first body.

In another embodiment of the second aspect, the method for expanding the bone further comprises allowing fluid to enter the internal cavity through the semi-permeable member to facilitate movement of the second body in the first direction relative to the first body. In yet another embodiment of the second aspect, the method for expanding the bone further comprising rotating a restrictor, positioned on the first side of the first body over the perforated cap, with respect to the perforated cap to at least partially block perforations of the perforated cap to at least partially restrict a flow of fluid into the internal cavity. In yet another embodiment of the second aspect, the method for expanding the bone further comprising translating a restrictor, positioned on the first side of the first body over the perforated cap, from a first orientation to a second orientation to block perforations of the perforated cap from fluid flow. The step of translating includes at least partially inserting a plurality of plugs of the restrictor into the perforations of the perforated cap, respectively, to block the perforations of the perforated cap from fluid flow.

A third aspect of the present disclosure includes an expandable bone implant system comprising a first body, a semi-permeable member, and a second body. The first body defines an internal cavity and a wall having perforations in communication with the internal cavity. The semi-permeable member is disposed adj acent to the perforations. The second body has a sealing end positioned within the internal cavity of the first body and a free end extending from a second side of the first body, the second body being moveable relative to the first body. The first body includes an osmotic chamber defined within the internal cavity, and the osmotic chamber is fluidly connected to the semi-permeable member such that fluid flow through the perforations from the semi-permeable member to the osmotic chamber in a first direction facilitates movement of the second body in the first direction relative to the first body. An outer surface of the first body includes a groove, and a portion of the second body disposed over the outer surface of the first body has depth stop apertures aligned with the groove, wherein a plug disposed in one of the depth stop apertures and in the groove defines a limit of displacement of the second body with respect to the first body, each of the depth stop apertures corresponding to a different limit of displacement.

In another embodiment of the third aspect, the perforations are confined to a location in the wall adjacent one side of the internal cavity. In yet another embodiment of the third aspect, an intramedullary nail comprises the first body and the second body. In yet another embodiment of the third aspect, at least one of the first body and the second body is curved.

A fourth aspect of the present embodiment includes a method for expanding a bone. A step of the method includes inserting a plug into a depth stop aperture extending along an outer surface of a second body, the plug extending from the depth stop aperture into a groove extending along an outer surface of a first body of the expandable bone implant system to define a limit of displacement of the second body with respect to the first body, the first body defining an internal cavity and the second body having a sealing end positioned within the internal cavity of the first body and a free end extending from a second side of the first body, the second body being moveable relative to the first body. Another step of the method includes anchoring the first body within the bone, the first body including a wall having perforations in communication with the internal cavity and an osmotic chamber defined within the internal cavity, the system further including a semi-permeable member disposed between the perforations and the internal cavity. Another step of the method includes anchoring the second body within the same bone. Another step of the method includes, after the anchoring steps, cutting the bone at the second side of the first body such that a first portion of the bone is connected to the first body and a separated second portion of the bone is connected to the second body, wherein the osmotic chamber is fluidly connected to the semi-permeable member such that fluid flow through the perforations from the semi-permeable member to the osmotic chamber in a first direction facilitates movement of the second body in the first direction relative to the first body.

In another embodiment of the fourth aspect, the method for expanding the bone further comprises allowing fluid to enter the internal cavity through the semi-permeable member to facilitate movement of the second body in the first direction relative to the first body. In yet another embodiment of the fourth aspect, the step of inserting the plug includes inserting the plug into one of a plurality of depth stop apertures each defining a different limit of displacement. In yet another embodiment of the fourth aspect, the steps of anchoring including anchoring the respective first and second bodies within a medullary canal of the bone.

A fifth aspect of the present disclosure includes an expandable bone implant system comprising a first body, a restrictor, a semi-permeable member, and a second body. The first body defines an internal cavity. The restrictor is configured to connect with a first side of the first body, wherein the restrictor includes a projection extending from an internal rod through an opening in an outer surface of the restrictor. The semi-permeable member is disposed adjacent to the restrictor. The second body has a sealing end positioned within the internal cavity of the first body and a free end extending from a second side of the first body, the second body being moveable relative to the first body. The first body includes an osmotic chamber defined within the internal cavity, and the osmotic chamber is fluidly connected to the semi-permeable member such that fluid flow through the opening from the semi-permeable member to the internal cavity in a first direction facilitates movement of the second body in the first direction relative to the first body.

In another embodiment of the fifth aspect, the rod extends through an internal channel adjacent to the internal cavity, and the rod is configured to move in a second direction different from the first direction. The internal channel is connected to the internal cavity by an opening such that fluid flows through the recess to the internal channel and through opening to the internal cavity. A first end of the rod includes a slot, and the rod is rotatable around an axis parallel to the second direction.

### BRIEF DESCRIPTION OF THE DRAWINGS

FIG. 1 is a perspective view of an expandable bone implant system according to an embodiment of the present disclosure.
FIG. 2 is another perspective view of the expandable bone implant system of FIG. 1.
FIG. 3 is another perspective view of the expandable bone implant system of FIG. 1.
FIG. 4 is a cross-sectional view of the expandable bone implant system of FIG. 1.
FIG. 5 is a perspective view of a restrictor of the expandable bone implant system of FIG. 1.
FIG. 6 is another perspective view of the restrictor of FIG. 5.
FIG. 7 is another perspective view of the restrictor of FIG. 5.
FIG. 8 is a perspective view of another embodiment of an expandable bone implant system.
FIG. 9 is a cross-sectional view of the expandable bone implant system of FIG. 8.
FIG. 10 is another cross-sectional view of the expandable bone implant system of FIG. 8.
FIG. 11 is perspective view of another embodiment of a restrictor of an expandable bone implant system.
FIG. 12 is another perspective view of the restrictor of FIG. 11.
FIG. 13 is another perspective view of the restrictor of FIG. 11.
FIG. 14 is another perspective view of the restrictor of FIG. 11.
FIG. 15 is a cross-sectional view of another embodiment of a first body of an expandable bone implant system.
FIG. 16 is a cross-sectional view of another embodiment of a first body of an expandable bone implant system.
FIG. 17 is a cross-sectional view of another embodiment of a first body of an expandable bone implant system.
FIG. 18 is a front plan view of an expandable bone implant system according to another embodiment of the present disclosure.
FIG. 19 is a side cross-sectional view of the expandable bone implant system of FIG. 18.
FIG. 20 is a perspective view of the expandable bone implant system of FIG. 18 implanted into a bone.

### DETAILED DESCRIPTION

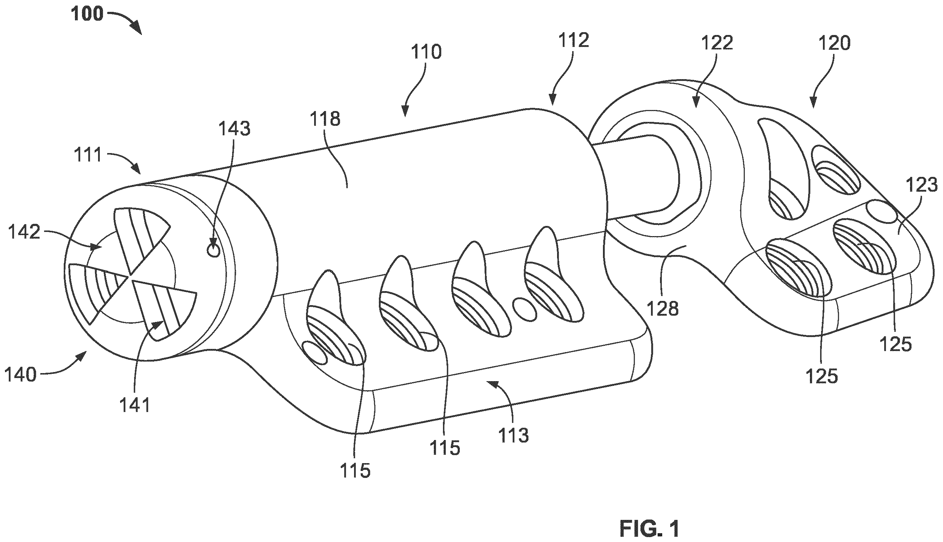

Referring to FIGS. 1-7, an expandable bone implant system 100 according to an embodiment of the present disclosure is shown. Implant system 100 is designed to be fixed to a bone by common fixation elements such as traditional or locking bone screws. Implant system 100 utilizes hydraulic technology to achieve expansion, as described in greater detail below. Thus, implant system 100 allows for initial distraction of a bone as well as continued expansion after the initial implantation to accommodate changing surgical parameters, including the growth of the patient.

Implant system 100 includes a first body 110 and a second body 120. First body 110 has a substantially cylindrical portion 118 of a circular profile. First body 110 also includes a first plate portion 113 with a plurality of apertures 115 for receiving fasteners to secure first body 110 to a bone. Similarly, second body 120 includes a substantially cylindrical portion 128 of a circular profile and a second plate portion 123 with a plurality of apertures 125 for receiving fasteners to secure second body 120 to the bone. The circular profiles can be different geometries in other examples, such as square, hexagon, or other irregular and/or non-circular shapes. First and second bodies 110, 120 and any other implanted components thereof may be formed of any surgical grade rigid materials suitable for implantation within a patient's body, such as plastic, ceramic, or metal such as titanium, titanium alloys, stainless steel, cobalt chrome alloys, tantalum and niobium, or any combination thereof. Gold and/or silver can be provided in the material composition or as a coating of a component. Each component of the present invention may be formed by an additive manufacturing process or 3D printing, including but not limited to electron beam melting (EBM), selective laser sintering (SLS), selective laser melting (SLM), binder jet printing, and blown powder fusion for use with metal powders.

First body 110 includes an internal cavity 114 that has a generally cylindrical shape with an osmotic chamber 117 defined within. Osmotic chamber 117 may include an osmotic agent, such as salt NaCl, as shown and described in U.S. Pat. No. 11,446,064, the entire disclosure of which is incorporated by reference herein. Osmotic chamber 117 is fluidly connected to a semi-permeable member or membrane 116 positioned near a first end or first side 111 of first body 110 that is opposite from a second end or side 112 of first body 110. Internal cavity 114 is configured to receive at least a first end or sealing end 121 of the second body 120. First end 121 of second body 120 is in the shape of a cylindrical rod or piston extending from a second end or free end 122 of second body 120.

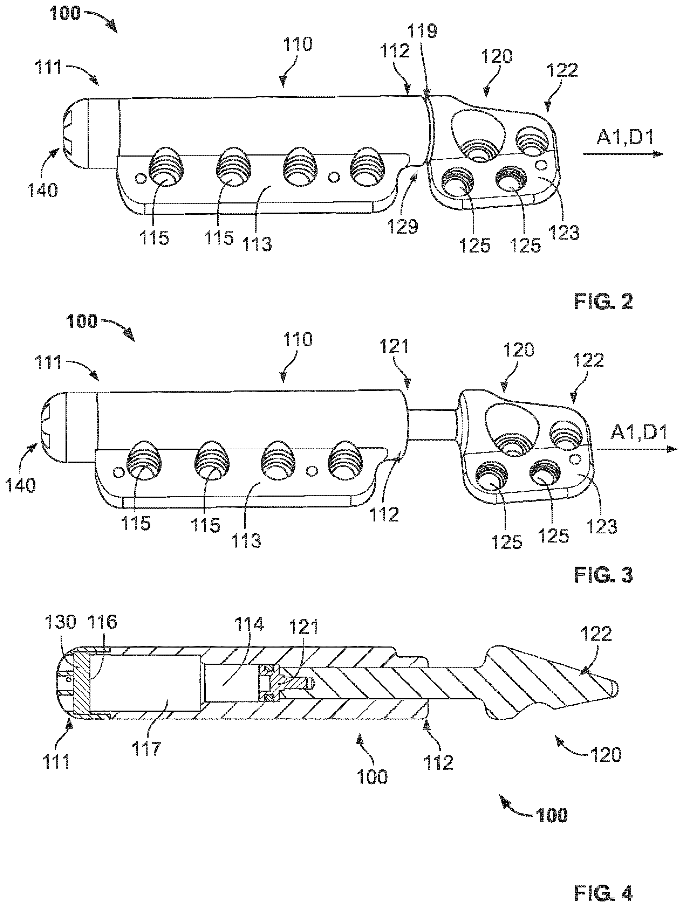

A perforated cap 130 is attached to first end 111 of first body 110 to enclose cavity 114 and is adjacent semi-permeable member 116. Cap 130 includes a plurality of perforations extending through first end 111 to provide access to osmotic chamber 117. Semi-permeable member 116 is disposed at least partially between cap 130 and internal cavity 114. Cap 130 and semi-permeable member 116 allow external fluid to enter into the osmotic chamber 117 and internal cavity 114, and semi-permeable member 116 is configured to prevent such fluid from exiting internal cavity 114. In that way, semi-permeable member 114 acts as a one-way valve. Accordingly, as a volume of fluid increases within the internal cavity 114, this volume creates a pressure within the internal cavity 114 since the fluid cannot escape, such that the fluid exerts a force on first end 121 of second body 120 causing second body 120 to move in a first direction or expansion direction D1 opposite the first body 110 along a longitudinal axis A1, as shown in FIGS. 2-3, extending through the first and second bodies 110, 120. A rate of expansion of second body 120 relative to first body 110 may vary or may remain constant over time until a predetermined expansion distance is met.

Positioned over the cap 130 at the first side 111 of first body 110 is a restrictor 140, as best shown in FIGS. 1 and 5-7. Restrictor 140 is circular in shape and includes a plurality of perforations 141 extending through an entire thickness of restrictor 140. Each of the perforations 141 are triangular in shape and separated by a solid surface 142 of restrictor 140. Perforations 141 are designed to match the shape of each of the perforations of the cap 130, though an exactly matching configuration need not be provided to allow system 100 to be effective.

Restrictor 140 includes an indent 143 along its peripheral edge. Indent 143 is configured to accommodate an end of a tool that can be used to engage indent 143 to rotate the restrictor 140 in either direction about longitudinal axis A1 in relation to cap 130, as previously discussed. Restrictor 140 may also include a projection 144 disposed within a recess 132 of cap 130. Projection 144 has a smaller width than the width of the recess 132, so the projection 144 can be moved between ends of the recess 132 to limit rotation of the restrictor 140 with respect to the perforated cap 130. Recess 132 includes three separate positions defined by individual indentations within recess 132 for the projection 144 to be fixed to the cap 130 which allows the restrictor 140 to be in three different orientations relative to the cap 130, as discussed below.

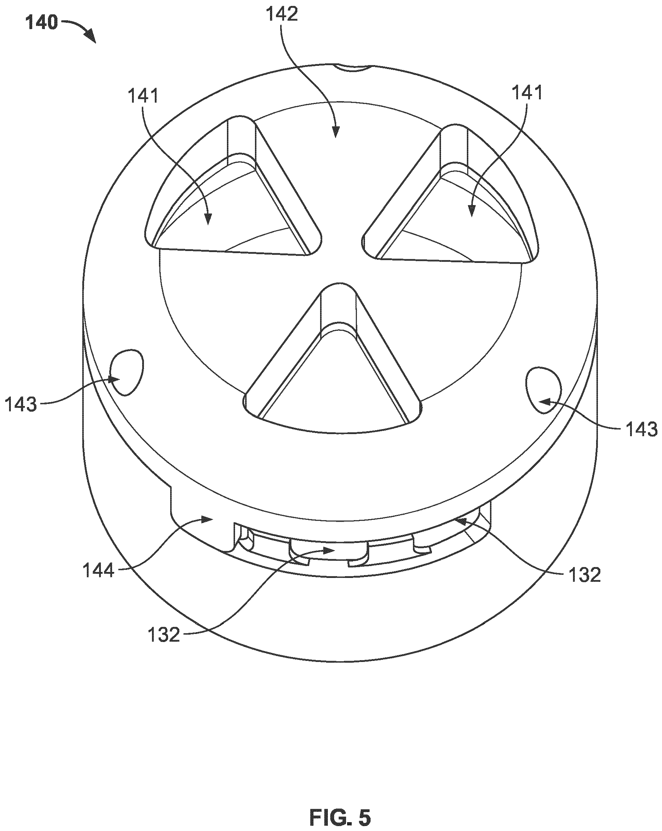

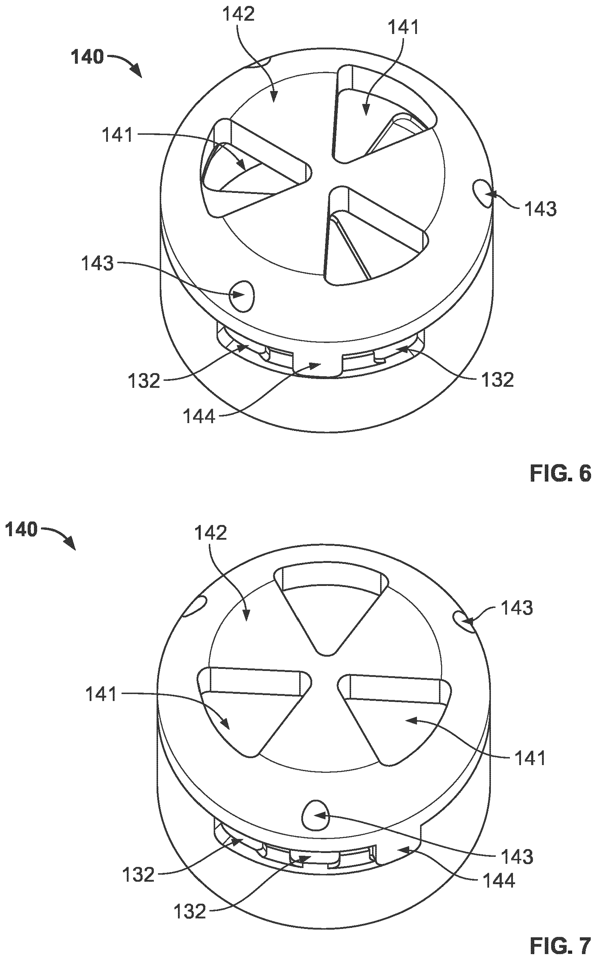

Restrictor 140 is configured to be rotatable about longitudinal axis A1 in order to position the plurality of perforations 141 in relation to the plurality of perforations of cap 130. In one orientation, shown in FIG. 5, rotation of restrictor 140 with respect to cap 130 aligns perforations 141 of restrictor with perforations of cap 130, and this allows a total cross-sectional area of the perforations of cap 130 to be exposed to fluid flow. In another orientation, shown in FIG. 6, rotation of restrictor 140 with respect to cap 130 at least partially blocks the perforations of cap 130 such that less than the total cross-sectional area of the perforations of the cap 130 is exposed to fluid flow. The total cross-sectional area of the perforations of the cap 130 exposed to fluid flow varies depending upon the rotational position of restrictor 140 and the total cross section are of the surface 142 of restrictor blocking the total cross-sectional area of the perforations of the cap 130. Thus, restrictor 140 can be moved in one direction to close access to internal cavity 114, shown in FIG. 7, and in another direction to open access to internal cavity 114 to control fluid flow through perforated cap 130.

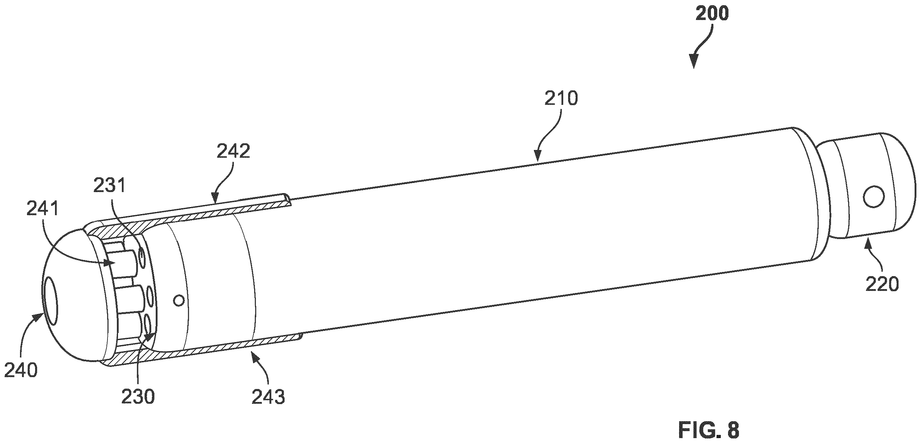

In another embodiment, as shown in FIGS. 8-10, an implant system 200 includes a first body 210 and a second body 220. System 200 is similar in many respects to system 100 with like elements being similarly numbered. First body 210 includes a restrictor 240 positioned over a perforated cap 230. Restrictor 240 and cap 230 differ from restrictor 140 and cap 130, as discussed below, but most other components of implant system 200 remain the same. First and second bodies 210, 220 are generally cylindrical and can be secured to bone segments by screws, cables, or the like, or can be secured to separate plate components for attachment to bone segments.

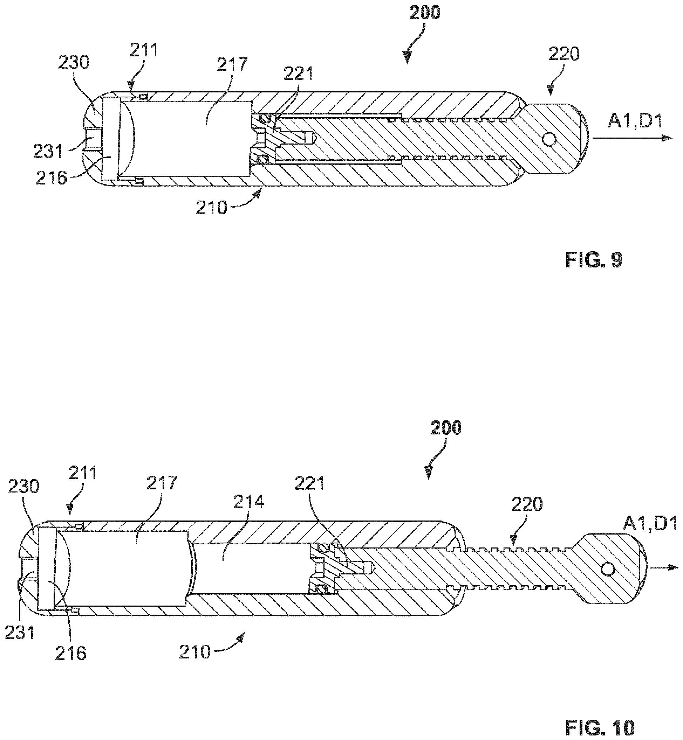

Cap 230 is attached to first end 211 of first body 210 and is adjacent semi-permeable member 216. Cap 230 includes a plurality of perforations 231 extending through first end 211 and into osmotic chamber 217. Semi-permeable member 216 is disposed at least partially between cap 230 and internal cavity 214. Cap 230 and semi-permeable member 216 allow fluid external to implant system 200 to enter into the osmotic chamber 217. As the volume of fluid increases within the internal cavity 214, this volume creates a pressure within the internal cavity that exerts a force on first end 221 of second body 220 causing the second body to move in a first direction or expansion direction D1 opposite the first body 210 along a longitudinal axis A1, as shown in FIGS. 9-10, extending through the first and second bodies 210, 220.

Restrictor 240 is circular in shape and includes a plurality of cylindrical plugs 241 extending from an inner surface of the restrictor 240 towards cap 230 along longitudinal axis A1 in the first direction D1. The plurality of plugs 241 are shaped and designed to be at least partially inserted in each of the plurality of perforations 231 of cap 230. Restrictor 240 also includes first and second prongs 242, 243 extending from a peripheral edge of restrictor 240 along longitudinal axis A1 in the first direction D1. Prongs 242, 243 are connected to an outer surface of first body 210, such as by fitting into corresponding recesses in the outer surface, to secure restrictor 240 to the first body 210 and further ensure a linear or axial translation of the components.

Restrictor 240 is configured to be translated along longitudinal axis A1 in the first direction D 1 in order to insert the plurality of plugs 241 into the plurality of perforations 231 of cap 230. In one orientation, the plurality of plugs 241 are not inserted into the plurality of perforations 231, thereby forming a gap or opening between the cap 230 and restrictor 240, allowing the plurality of perforations 231 to be exposed to fluid flow. In another orientation, the plurality of plugs 241 are at least partially inserted into the plurality of perforations 231, thereby blocking the plurality of perforations 231 from being exposed to fluid flow. In other embodiments, each of the plugs 241 may be a different height so that some enter the plurality of perforations 231 of cap 230 before others, creating an opportunity to set different rates between open and closed. In still other embodiments, some or all of the plugs 241 can be cone shaped so that they enter but do not completely seal a perforation 231 of cap 230, rather reducing the cross-sectional area through which fluid can flow through cap 230.

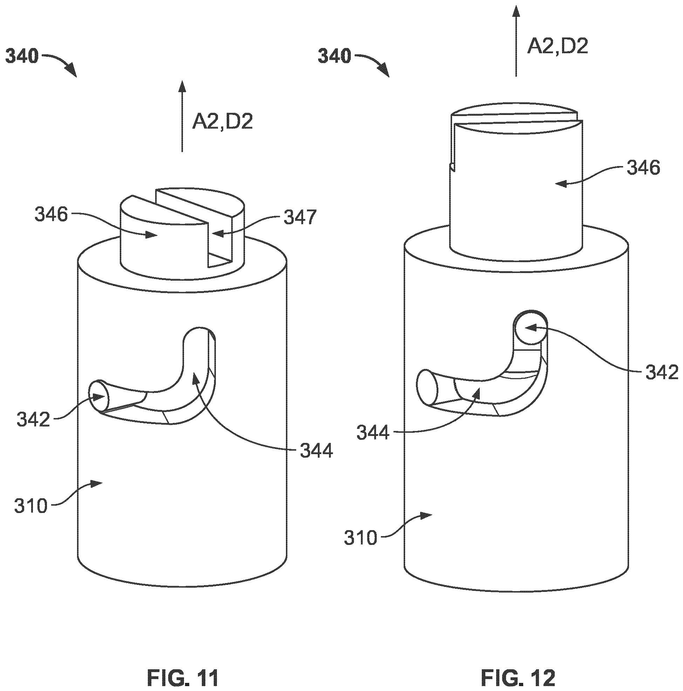

In another embodiment, as shown in FIGS. 11-14, an implant system 300 includes a first body 310 and a second body. System 300 is similar in many respects to systems 100, 200 with like elements being similarly numbered. First body 310 includes a restrictor 340 that differs from restrictors 140, 240, as discussed below, but most other components of implant system 300 remain the same. First body 310 and second body are generally cylindrical and can be secured to bone segments by screws, cables, or the like, or can be secured to separate plate components for attachment to bone segments.

Restrictor 340 includes a projection 342 extending from a cylindrical rod 346 through an opening 344 of the first body 310. Restrictor 340 allows external fluid to enter through the opening 344 into an internal channel 348 of restrictor 342. As fluid enters into the internal channel 348, it then flows through an opening 349 into an internal cavity 314 of first body 310. As previously discussed for systems 100, 200, an osmotic chamber is defined within internal cavity 314, and a semi-permeable member is disposed at least partially between the opening 344 and the internal cavity 314. Accordingly, as a volume of fluid increases within the internal cavity 314, this volume creates a pressure within the internal cavity 314 since the fluid cannot escape, such that the fluid exerts a force on first end of second body causing second body to move in a first direction or expansion direction opposite the first body 310 along a longitudinal axis, extending through first body 310 and second body. A rate of expansion of second body relative to first body 310 may vary or may remain constant over time until a predetermined expansion distance is met.

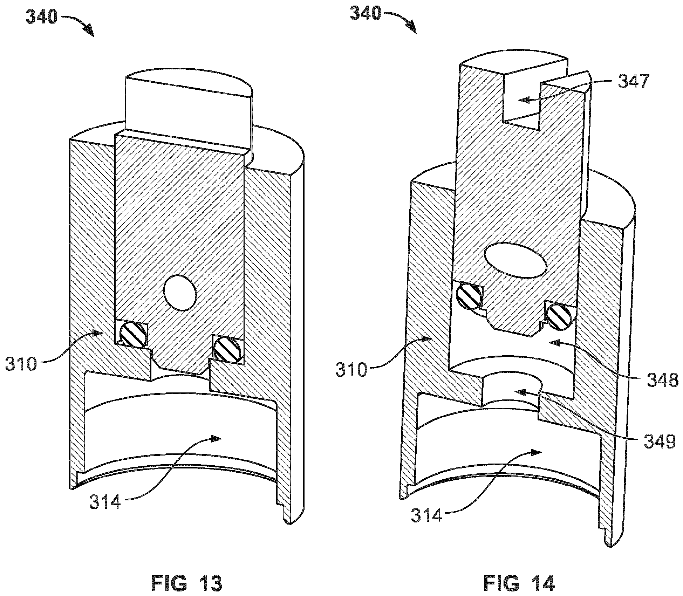

Projection 342 of restrictor 340 is configured to be moveable within the opening 344 in order to control the flow of external fluid into the internal cavity 314. In one orientation, shown in FIGS. 11 and 13, projection 342 is in a first position causing rod 346 to be positioned within internal channel 348 to fully block external fluid from entering through the opening 344 and into the internal channel 348. In another orientation, shown in FIGS. 12 and 14, projection 342 is in a second position causing rod 346 to be moved within internal channel 348 along a longitudinal axis A2 in a second direction D2 opposite the second body to create an opening for external fluid to enter through the opening 344 into the internal channel 348. Thus, projection 342 can be moved to one position, such as via rotation of a tool like a flathead screwdriver in a slot 347 at an end of rod 346 of restrictor 340, to close access to internal channel 348 and internal cavity 314, shown in FIGS. 11 and 13, and in another direction to open access to internal channel 348 and internal cavity 314 through the opening 344, shown in FIGS. 12 and 14.

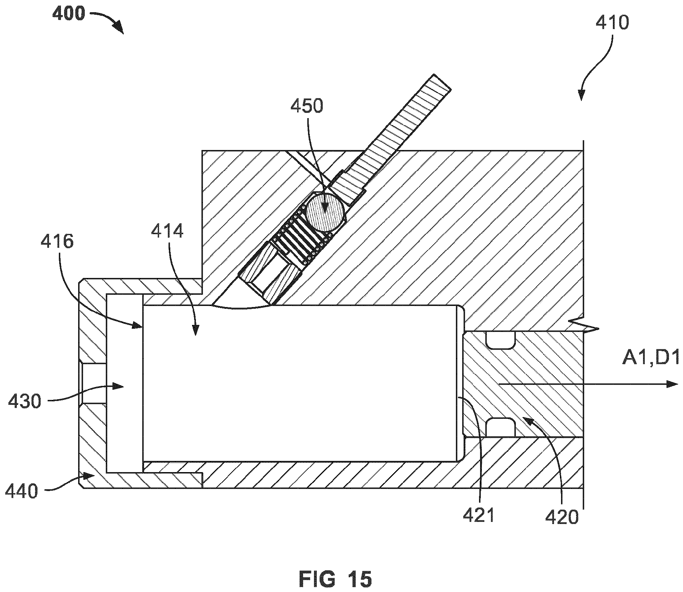

In another embodiment, as shown in FIG. 15, first body 410 of implant system 400 includes a valve 450. Valve 450 is disposed in an opening or channel extending from internal cavity 414 through first body 410. All other components of implant system 400 remain the same in comparison to system 100, and valve 450 can be used in combination with any of restrictors 140, 240, 340 and caps 130, 230 previously discussed herein.

Valve 450 is moveable in a direction away from the osmotic chamber and internal cavity 414, and this movement of the valve is configured to release pressure built up within the osmotic chamber and internal cavity 414. As previously discussed, restrictor 440, cap 430, and semi-permeable member 416 allow fluid external to implant system 400 to enter into the osmotic chamber. As the volume of fluid increases within the internal cavity 414, this volume creates a pressure within the internal cavity that exerts a force on first end 421 of second body 420 causing the second body 420 to move in a first direction or expansion direction D1 opposite the first body 410 along a longitudinal axis A1, as shown in FIGS. 15-17, extending through the first and second bodies 410, 420. Valve 450 is configured to reduce the pressure within the internal cavity 414 and osmotic chamber to reduce the force on first end 421 of second body 420. The release of pressure by the valve 450 may either reduce the rate of expansion of the second body 420 or may stop the expansion of the second body all together.

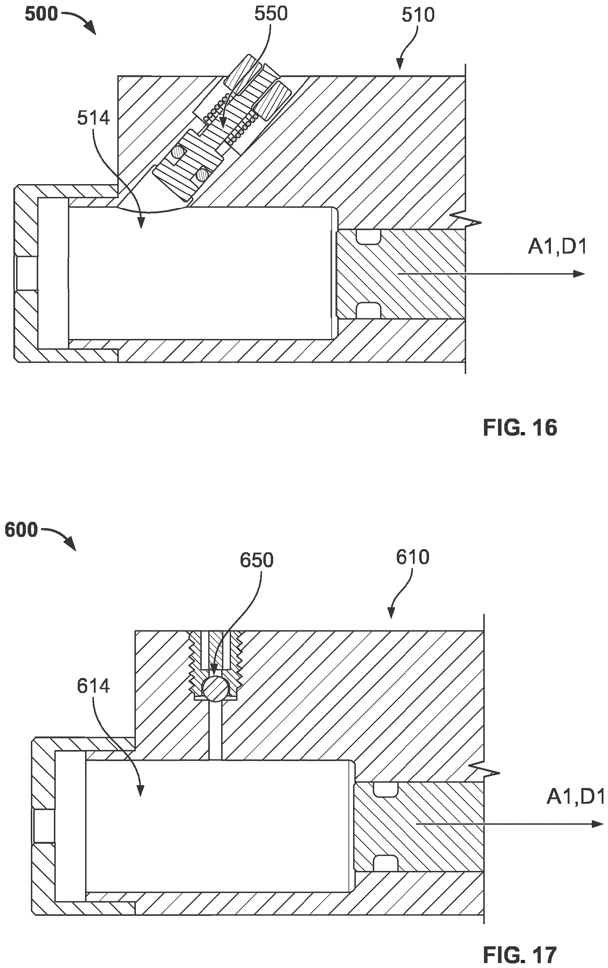

In other embodiments, as shown in FIGS. 16-17, first body 510 of implant system 500 includes a valve 550 and first body 610 of implant system 600 includes a valve 650. Valves 550, 650 are configured to interact with respective internal cavities 514 and 614 and respective first bodies 510, 610 in the same manner as valve 450. Valves 550, 650 differ from valve 450 only in the internal components and design of each respective valve.

A method for expanding a bone is performed using implant system 100. While implant system 100 may be used to expand a long bone such as a femur, tibia, or humerus, other bones for which lengthening is desired can also utilize system 100. The method for expanding a bone use the implant system 100 may be for initial distraction of the bone as well as continued expansion after the initial implantation to accommodate changing surgical parameters, including the growth of the patient. While system 100 is mainly used in the description below, the other aforementioned systems may also be used by the same or similar methods when accounting for the variations in structure described above.

The first and second bodies 110, 120 of implant system 100 are anchored onto the bone of the patient. First and second bodies 110, 120 are designed to be fixed to the bone by common fixation elements such as bone screws, locking screws, or the like. After the first and second bodies 110, 120 are anchored and secured to the bone, the bone is then cut at a location that aligns with a mating surface connection 119 between the second end 112 of the first body 110 and the first end 121 of the second body 120, as shown in FIG. 2. To facilitate the step of cutting, a gap 129 is provided between first plate portion 113 and second plate portion 123 to create space at the bone for cutting instruments. As shown in FIG. 2, gap 129 is achieved by a removed or recessed portion of first plate portion 113, though gap 129 may also be provided by a removed or recessed portion of second plate portion 123 or by removed sections of both plate portions 113, 123.

In this way, the proper anatomical alignment of the bone is preserved by securing system 100 to the bone before cutting. That is, a perfect bone alignment of the two bone segments is achieved. System 100 is aligned so that its expansion direction D1 is aligned to be parallel to the longitudinal axis of the bone. Once the bone is cut, the segments of the bone can separate along an axis that is parallel to the natural bone axis, which maintains the natural alignment of the bone after lengthening. The bone may be cut by any commonly used method or technique. One method that compliments the presence of system 100 on the bone is the use of an oscillating wire looped behind the bone and away from system 100. The wire can be oscillated to cut the bone behind mating surface connection 119 until the wire reaches system after cutting through the extent of the bone. After the bone is cut, first body 110 remains anchored or secured to a first portion of bone and second body 120 remains anchored or secured to a distinct second portion of the same bone.

As fluid within the body of the patient that is external to implant system 100 is allowed to enter into internal cavity 114 and osmotic chamber 117 through semi-permeable member 116, pressure builds within internal cavity 114 to facilitate movement of second body 120 in the first direction relative to the first body 110. In other words, as the volume of fluid increases within the internal cavity 114, this volume exerts a force on first end 121 of second body 120 causing the second body to move in a first direction or expansion direction D1 opposite the first body 110 along a longitudinal axis A1 extending through the first and second bodies 110, 120, as shown in FIGS. 2-3. The second portion of the bone secured to the second body 120 moves away from the first portion of the bone secured to the first body 110, and thereby the distance in between the first and second portions of the bone increases. Thus, system 100 is operable on its own after implantation through use of fluid located within the body of the patient. The rate at which fluid enters osmotic chamber 117 can be manipulated by adjusting the parameters of semi-permeable member 116 such as its constitution, density, and/or thickness.

A further ability to manipulate the rate at which fluid enters into osmotic chamber 117 is permitted by adjustment of restrictor 140 with respect to cap 130 in order to at least partially restrict the flow of fluid into the osmotic chamber 117 and internal cavity 114. Rotation of restrictor 140 about longitudinal axis A1 with respect to cap 130 at least partially blocks the perforations of cap 130 to at least partially restrict the flow of fluid into the osmotic chamber 117 and internal cavity 114. The rotational position of restrictor 140 with respect to cap 130 can be done prior to, during, or after initial implantation of system 100. Thereafter, restrictor 140 can be adjusted by, for example, inserting an end of a tool into indent 143 of restrictor to manipulate it by rotating it with respect to cap 130. This will change the alignment of the perforations of restrictor 140 with respect to the perforations in cap 130, either to increase or decrease the cross-sectional open area through which fluid can flow into cavity 114. The projection 144 of restrictor 140 within the recess 132 of cap 130 provides end stops for completely opening or completely closing the cross-sectional area. This may be done postoperatively to either increase or slow, and even to stop, the rate of elongation of system 100. One may stop elongation if enough distraction has occurred. Otherwise, a rate change could be determined to be necessary based on the growth rate of the patient, which can vary over time. A postoperative adjustment of restrictor 140 can be done through a minimally invasive surgical procedure using a relatively small incision to simply permit access to restrictor beneath the skin and tissue of the patient. In some embodiments, once extension is complete and sufficient bone growth occurs to bridge the gap formed between the separated bone segments, a procedure may be conducted to remove system 100.

When using system 200, adjustment of restrictor 240 with respect to cap 230 includes translating restrictor 240 along longitudinal axis A1 in the first direction D1 from a first orientation to a second orientation to block the flow of fluid through the perforations 231 in cap 230 into the osmotic chamber 217 and internal cavity 214. This translation includes at least partially inserting the plurality of plugs 241 of restrictor 240 into the plurality of perforations 231, respectively, of cap 230 to block the flow of fluid. As indicated above in connection with system 100, this can be done prior to, during, or after the procedure, the latter by way of a minimally invasive surgical procedure.

When using system 300, adjustment of restrictor 340 includes rotating and translating rod 346 to move projection 342 from a first orientation to a second orientation to allow the flow of fluid through the opening 344 into the internal channel 348 and internal cavity 314. This translation moves projection 342 along the opening 344 as the rod 346 moves along longitudinal axis A2 in the second direction D2. As indicated above in connection with system 300, this can be done prior to, during, or after the procedure, the latter by way of a minimally invasive surgical procedure.

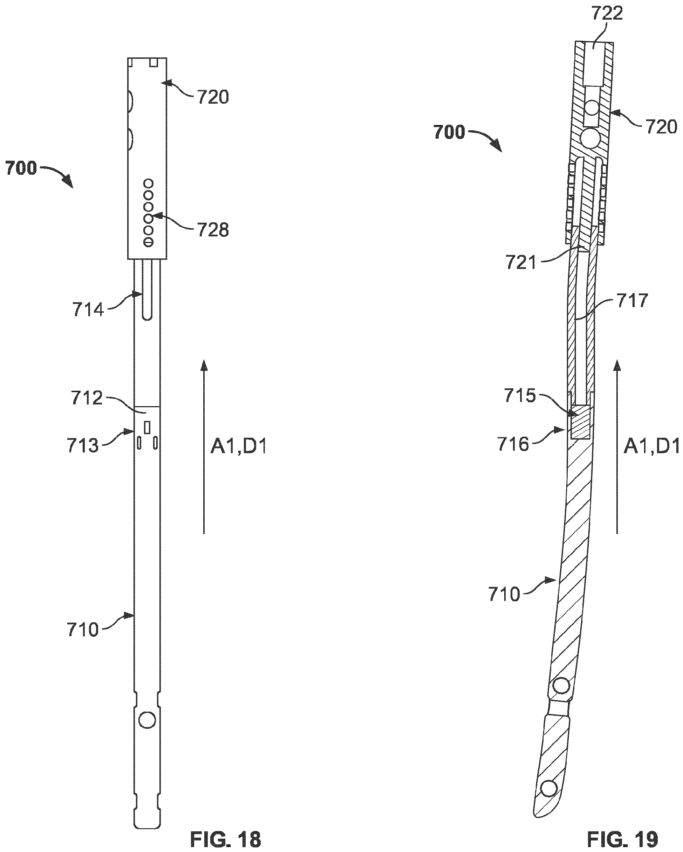

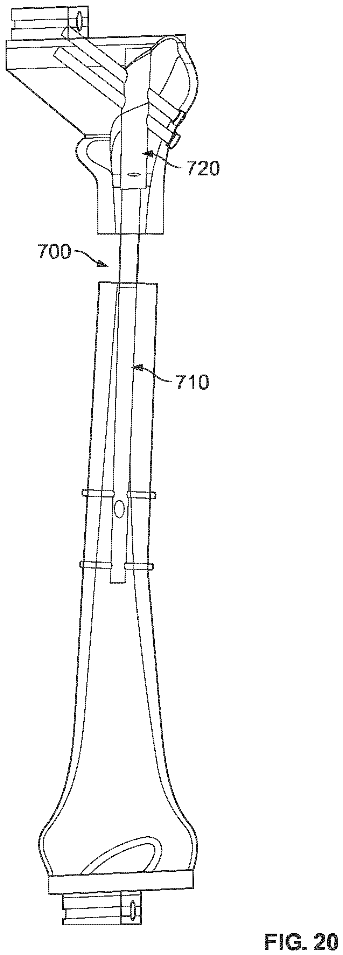

Now referring to FIGS. 18-20, an expandable bone implant system 700 according to another embodiment of the present disclosure is shown. Implant system 700 includes a first body 710 and a second body 720 that form portions of a self-distracting intramedullary nail for implantation within a long bone for correction of a deformity and/or stabilization of a bone while allowing lengthening of the bone during growth. System 700 can be said to include only first and second bodies 710, 720, and alternately can include any other portions necessary to assemble the complete self-distracting intramedullary nail. As best shown in FIG. 20, first body 710 and second body 720 each include a plurality of apertures for receiving fasteners to secure the implant system within a bone, where first body 710 is secured to a first portion of bone and second body 720 is secured to a second portion of bone. Additionally, either or both of first body 710 and second body 720 may be entirely curved or have at least a portion that is curved to match the geometry of the bone or portions of bone. With this it might be possible to correct a malalignment, for example varus or valgus of the lower extremities. The lengthening along a curved axis is beneficial over prior devices that may be confined to purely linear expansion along a straight axis due to threaded engagement between the parts.

First body 710 includes an internal cavity 716 that is cylindrical shape and has osmotic chamber 717 defined within. Internal cavity 716 is in a proximal center position of first body 710. Internal cavity 716 is configured to receive at least a first end or sealingly end 721 of the second body 720. First end 721 of second body 720 is in the shape of a cylindrical rod extending from a position proximal to the center of second body 720. Second body 720 includes a second end or free end 722 opposite first end 721.

Extending through an outer surface or wall of first body 710 and confined to a location in the wall adjacent one side of the internal cavity 716 is a plurality of perforations 713. A semi-permeable member 715 is disposed at least partially between perforations 713 and internal cavity 716. Perforations 713 and semi-permeable member 715 allow fluid external to implant system 700, i.e., fluid within an intramedullary canal of a bone in which the nail is inserted, to enter into the osmotic chamber 717 and internal cavity 716. As a volume of fluid increases within the internal cavity 716, this volume creates a pressure within the internal cavity that exerts a force on first end 721 of second body 720 causing the second body to move in a first direction or expansion direction D1 opposite the first body 710 along a longitudinal axis A1 extending through the first and second bodies 710, 720, as shown in FIGS. 18-19.

The outer surface or wall of first body 710 includes a groove 714 extending along the longitudinal axis A1 and positioned near a first end 611 of first body 710. A portion of an outer surface or wall of second body 720 is configured to be positioned over groove 714 of first body 710 when first and second bodies 710, 720 are assembled as shown in FIGS. 18-19. The portion of second body 720 aligned with groove 714 includes a plurality of depth stop apertures 728 extending through second body 720 and permitting access to groove 714.

All of the apertures 728 are configured to receive a plug that can be inserted into groove 714 to dictate the extent of movement of second body 720 relative to first body 710. Therefore, the aperture 728 that receives plug defines a limit of displacement of the second body 720 relative to first body 710 as it moves in the first direction D1 along the longitudinal axis A1. In other words, a plug in a relatively lower aperture 728 (i.e., closer to first body 710) as shown in FIGS. 18-19 will permit nail to extend to a greater overall length since the plug will not encounter the upper end of groove 714 until second body 720 is further away from first body 710. For the same reason, a plug in a relatively higher aperture 728 (i.e., further from first body 710) as shown in FIGS. 18-19 will maintain a relatively shorter greater overall length of the nail since second body 720 cannot extend as far away from first body 710. In that way, each of the depth stop apertures 728 corresponds to a different limit of displacement that can be set by a surgeon prior to implantation of system 700. A rate of expansion of second body 720 relative to first body 710 may vary or may remain constant over time until the limit of displacement is reached.

A method for expanding a bone using implant system 700 uses system 700 as an intramedullary nail to expand a long bone such as a femur or tibia. The method for expanding a bone use the implant system 700 may be for initial distraction of the bone as well as continued expansion after the initial implantation to accommodate changing surgical parameters, including the growth of the patient.

Prior to anchoring the implant system 700 into the bone, a plug is inserted into a depth stop aperture 728 of the second body 720. If more than one groove 714 and set of depth stop apertures 728 are present, this can be done multiple times as necessary around the various sides of the nail. The plug extends from the depth stop aperture 728 into grove 714 of first body 710 to define the limit of displacement of second body 720 with respect to first body 710. As indicated above, each individual aperture 624 defines a different limit of displacement of second body 720 relative to first body 710 so that a desired displacement can be set prior to insertion of implant system 700 into the bone.

Then, the first and second bodies 710, 720 of implant system 700 are anchored into the bone of the patient. The first and second body 710, 720 are designed to be implanted in a medullary canal of bone and are fixed to the bone by common fixation elements such as bone screws, locking screws, or the like. After the first and second bodies 710, 720 are anchored and secured within the bone, the bone is cut, for example at a location in between the second end 712 of the first body 710 and the first end 721 of the second body 720, though other locations are suitable so long as first body 710 is anchored within the first portion of the bone and second body 720 is anchored within the second portion of the bone. The bone may be cut by any commonly used method or technique, as discussed above. After the bone is cut, first body 710 remains anchored or secured to a first portion of bone and second body 720 remains anchored or secured to a second portion of bone.

As previously discussed, perforations 713 and semi-permeable member 715 of first body 710 allow fluid external to implant system 700 to enter into the osmotic chamber 717 and internal cavity 716. As a volume of fluid increases within the internal cavity 716, this volume creates a pressure within the internal cavity that exerts a force on first end 721 of second body 720 causing the second body to move in a first direction or expansion direction D1 opposite the first body 710 along a longitudinal axis A1, as shown in FIGS. 18-19, extending through the first and second bodies 710, 720. Since the cavity 716 is completely filled with fluid upon full expansion, the fully expanded length of the nail cannot be shortened, which permits permanent implantation without risk of failure, particularly as new bone growth bolsters the strength of the bone.

Although the invention herein has been described with reference to particular embodiments, it is to be understood that these embodiments are merely illustrative of the principles and applications of the present invention. It is therefore to be understood that numerous modifications may be made to the illustrative embodiments and that other arrangements may be devised without departing from the scope of the present invention as defined by the appended claims.

## Claims

1. An expandable bone implant system comprising:
a first body defining an internal cavity;
a perforated cap having perforations and configured to attach to a first side of the first body;
a semi-permeable member disposed adjacent to the perforations of the perforated cap;
a second body having a sealing end positioned within the internal cavity of the first body and a free end extending from a second side of the first body, the second body being moveable relative to the first body; and
a restrictor configured to control fluid flow through the perforations of the perforated cap,
wherein the first body includes an osmotic chamber defined within the internal cavity, and the osmotic chamber is fluidly connected to the semi-permeable member such that fluid flow through the perforated cap from the semi-permeable member to the osmotic chamber in a first direction facilitates movement of the second body in the first direction relative to the first body.

2. The expandable bone implant system of claim 1, wherein the restrictor is positioned on the first side of the first body over the perforated cap.

3. The expandable bone implant system of any one of claims 1 or 2, wherein the restrictor is rotatable about an axis parallel to the first direction.

4. The expandable bone implant system of any one of claims 1-3, wherein the restrictor includes perforations configured to align with the perforations of the perforated cap.

5. The expandable bone implant system of any one of claims 1-4, wherein the restrictor is configured to be aligned in a first orientation with respect to the perforated cap in which substantially all of a total cross-sectional area of the perforations of the perforated cap is exposed to fluid flow, in a second orientation with respect to the perforated cap in which the restrictor at least partially blocks the perforations of the perforated cap such that less than the total cross-sectional area of the perforations of the perforated cap is exposed to fluid flow, and in a third orientation with respect to the perforated cap in which the restrictor substantially blocks the perforations of the perforated cap such that the total cross-sectional area of the perforations of the perforated cap is substantially blocked to fluid flow.

6. The expandable bone implant system of any one of claims 1-5, wherein the restrictor defines an indent in a peripheral edge configured to provide access for a tool to rotate the restrictor with respect to the perforated cap.

7. The expandable bone implant system of any one of claims 1-6, wherein the restrictor includes a projection disposed within a recess of the perforated cap, the projection having a width smaller than a width of the recess such that the projection can be moved between ends of the recess to limit rotation of the restrictor with respect to the perforated cap.

8. The expandable bone implant system of any one of claims 1-7, wherein the restrictor is moveable in the first direction and in a second direction opposite the first direction.

9. The expandable bone implant system of any one of claims 1-8, wherein the restrictor includes a plurality of plugs respectively aligned with the perforations of the perforated cap.

10. The expandable bone implant system of claim 9, wherein the restrictor is configured to be translated from a first orientation with respect to the perforated cap in which the perforations of the perforated cap are substantially exposed to fluid flow to a second orientation with respect to the perforated cap in which the plurality of plugs are at least partially inserted into the perforations, respectively, to substantially block the perforations of the perforated cap from fluid flow.

11. The expandable bone implant system of any one of claims 1-10, wherein the first body further includes a valve configured to release pressure from the internal cavity.

12. The expandable bone implant system of any one of claims 1-11, wherein the first body includes a first plate portion and the second body includes a second plate portion, the first and second plate portions separated by a gap located adjacent the second side of the first body.

13. The expandable bone implant system of claim 12, wherein the first and second plate portions each include apertures through which fasteners can be inserted to secure the first and second plate portions to respective bone portions.
